# EUROPEAN PATENT SPECIFICATION

(11) **EP 3 841 995 B1**
(45) Date of publication and mention of the grant of the patent: **01.05.2024**
(21) Application number: 19896735.8
(22) Date of filing: 06.12.2019
(51) Int. Cl.: A61B 18/12

(54) **POSITION LIMITING DEVICE FOR A SURGICAL DEVICE AND METHOD FOR USING SAME**
POSITIONSLIMITIERENDE VORRICHTUNG FÜR EIN CHIRURGISCHES INSTRUMENT UND VERFAHREN ZU DEREN VERWENDUNG
DISPOSITIF LIMITANT LA POSITION D'UN DISPOSITIF CHIRURGICAL ET SON PROCÉDÉ D'UTILISATION

(30) Priority: 14.12.2018 CN 201811530424
(43) Date of publication of application: 30.06.2021
(73) Proprietor: Micro-Tech (Nanjing) Co., Ltd., Nanjing, Jiangsu 210032 (CN)
(72) Inventor: LI, Xiaochun, Nanjing, Jiangsu 210032 (CN); XU, Chaowei, Nanjing, Jiangsu 210032 (CN); LI, Changqing, Nanjing, Jiangsu 210032 (CN); LENG, Derong, Nanjing, Jiangsu 210032 (CN); LIU, Chunjun, Nanjing, Jiangsu 210032 (CN)
(74) Representative: Mitola, Marco
(86) International application number: PCT/CN2019/123493
(87) International publication number: WO 2020/119585

(56) References cited:
- CN-A- 109 512 505
- CN-U- 201 674 758
- CN-U- 201 674 758
- CN-U- 201 674 758
- CN-U- 202 801 543
- CN-U- 204 723 173
- CN-U- 205 658 989
- CN-U- 209 751 210
- DE-U1- 20 218 876
- JP-B2- 5 820 460
- US-A- 5 599 300
- US-A1- 2012 296 327

## Description

### Technical Field

The present disclosure relates to a medical instrument, in particular to a position limiting device in a surgical instrument according to claim 1.

### Background Art

Endoscopic retrograde cholangiopancreatography (ERCP) refers to a technology of inserting a duodenoscope into a descending part of duodenum to find duodenal papilla, inserting a radiography conduit to an opening portion of the papilla through a biopsy channel, and injecting a contrast agent and then performing X-ray radiography so as to display a pancreatic bile tract. The ERCP is well received by patients as it does not need invasive surgery, and has a small wound, short surgical time, less complication than surgery, and greatly shortened hospitalization period. Papilla incision is usually used for pancreatic bile tract system cannula and endoscopic sphincteropapillotomy (EST). EST is a therapeutic technology of partially incising duodenal papilla sphincter and common bile duct terminal using a high-frequency electric sphincterotome under an endoscope, further developed on the basis of the diagnosis technology of ERCP.

Clinically, EST is widely used in the treatment of diseases such as choledocholithiasis, biliary pancreatitis, acute obstructive suppurative cholangitis, periampullary tumor, ascaris of biliary tract, common bile duct terminal benign stricture, and Oddi sphincter dysfunction. After implementing endoscopic sphincteropapillotomy, diseases may be partially or completely treated in combination with stone breaking, calculus removing, ascaris removing, nasobiliary drainage, and stent drainage and so on.

By inserting an insulated guide wire into the common bile duct, a negative plate of a high-frequency electric generator is stuck on the skin of buttock of a patient, a connecting joint of a control handle of the sphincterotome is connected with a corresponding electrode joint of a high-frequency electric generator, and the incision is carried out by adjusting the electric incision and electric coagulation powers, and a mixing ratio thereof. Simple electrotomy current may also be used for EST, and it may reduce the coagulation effect on nearby tissues during the incision process, thereby being beneficial to maintain a clear endoscopic field of view.

In the conventional ERCP surgery, a papilla sphincterotome generally needs to perform an electric incision operation after the pancreaticobiliary duct system intubation is completed. Before the electric incision, a ring slider on the handle needs to be pulled to enable cutting wire at a head end to present a proper bow shape, so that the cutting wire is tightly attached to a papilla position which is preset to be electrically cut, and the cutting wire needs to continuously keep in the bow shape in this process.

There is a rapid-exchange sphincterotome product with a self-locking function on the handle currently in the market, after the position of the handle is adjusted, the sphincterotome stays at a preset position by means of friction force between the cutting wire and cutting wire cavity of outer tube, but due to the presence of the friction force between the cutting wire and the cutting wire cavity of outer tube, a large force is needed for pulling the handle, and the handle may be moved under the action of force.

Therefore, there is an urgent need to design a limiting device, which not only can realize the function of fast moving and positioning, but also still can realize a limiting function after a long-time placement.

### Summary

US 2012/0296327 A1 discloses an apparatus for, and a method of, accurate positioning of endoscopic instruments.

In view of the preceding deficiencies in the prior art, the present disclosure provides a limiting device, which limits a position mainly through cooperation with a core rod. When the position of the core rod is limited, the core rod itself may be limited, and its movement relative to the limiting device is limited, thus achieving the positioning effect; other parts on the core rod also may be limited, and their movement on the core rod is limited. Before use, there is no clamping force between the limiting device and the core rod in cooperation therewith, and the clamping force thereof in use still will not be affected even if after a long-time placement; when the limiting device needs to slide to limit position, the limiting device is adjusted to a designated position, and as there is no clamping force between the limiting device and the core rod at this time, the limiting device may slide freely on the core rod, to enable it to reach the designated position; when the limiting device is moved to the designated position, the limiting device is pressed, so that it is clamped tightly on the core rod, at this time, the position limiting function can be realized, when the position does not need to be limited, the limiting device may be adjusted to a non-clamping state, so that the movable portion cooperates with the core rod, then the position of the limiting device may be conveniently adjusted. The present disclosure will obviously improve the position limiting effect, prolong the service life of the limiting device, can facilitate the operation, and save the operation time.

In order to achieve the above objectives of the present disclosure, the technical means adopted in the present disclosure is to design a limiting device, wherein the limiting device includes a clamping portion and a movable portion, the clamping portion and the movable portion are both in a ring structure, two ends of the clamping portion in a ring structure and two ends of the movable portion in a ring structure are connected, respectively, forming a connected double-ring structure; a ring diameter of the movable portion is greater than a ring diameter of the clamping portion; the limiting device is provided on a core rod, the clamping portion and the core rod are in an interference fit, and the movable portion and the core rod are in a clearance fit; when the movable portion cooperates with the core rod, the limiting device can slide with respect to the core rod, when the clamping portion cooperates with the core rod, the limiting device and the core rod are in a fixed state, and the limiting device cannot move with respect to the core rod, thus achieving a position limiting purpose.

The advantages of the present disclosure lie in that the clamping portion and the movable portion are ring structures, before use or when clamping is not needed, the movable portion is enabled to cooperate with the core rod. As the movable portion and the core rod are in a clearance fit, the movable portion may freely slide on the core rod without generating a pressure, and generates no pressure on the limiting device when not being used, thus prolonging the service life of the limiting device. When the limiting device needs to be used to limit the position, the limiting device may be conveniently slid without applying a large force, to achieve the purpose of facilitating the operation; when the limiting device is needed to limit the position, the limiting device is pressed, to enable the clamping portion to cooperate with the core rod. As the clamping portion and the core rod are in an interference fit therebetween, when the clamping portion cooperates with the core rod, the limiting device will not slide any more, thus achieving the purpose of clamping and position limiting.

Preferably, the core rod is further provided with a slider, when the slider moves to a designated position, to enable the clamping portion to cooperate with the core rod, the limiting device and the core rod are in a fixed state, so that the limiting device limits movement of the slider.

Preferably, when the clamping portion and the movable portion cooperate with the core rod, an area of the core rod wrapped by the clamping portion and the movable portion is greater than half of a ring area of the core rod. When the clamping portion and the movable portion cooperate with the core rod, and the part of the core rod wrapped by the clamping portion is greater than half of the ring area of the core rod, the pressure at a connecting part of the movable portion and the clamping portion can be reduced, the clamping portion of the limiting device is prevented from sliding off the core rod, thus achieving a better fixing effect.

Preferably, the clamping portion is provided with a pressing plate, and the core rod is provided with a groove cooperating with the pressing plate. In order to achieve a better clamping effect, the clamping portion in the present disclosure is provided with the pressing plate, which can be embedded into the groove on the core rod, thus increasing the pressure in the clamping state.

Preferably, the pressing plate is of a fork shape or a zigzag shape.

Preferably, the depth of the groove is greater than the length of the pressing plate.

Preferably, when the movable portion cooperates with the core rod, the limiting device is in a non-clamping state, only end portions of the pressing plate are in contact with the groove of the core rod; and when the clamping portion cooperates with the core rod, the limiting device is in a clamping state, the pressing plate is mostly or completely pressed into the groove of the core rod, and the limiting device cannot move with respect to the core rod.

Preferably, the clamping portion and the movable portion are integrally molded or separately molded.

Preferably, the ring structures of the clamping portion and the movable portion are C-shaped structures.

Preferably, a material of the limiting device is plastic.

The present disclosure further provided a method for using a limiting device. When the limiting device is in a non-clamping state, the limiting device is slid to a designated portion; the clamping portion of the limiting device is pressed, so that the clamping portion of the limiting device cooperates with the core rod, at this time, the limiting device is in a clamping state; after the position limiting is completed, the movable portion of the limiting device is pressed, so that the movable portion of the limiting device cooperates with the core rod, the limiting device is again in a non-clamping state, avoiding the core rod and the limiting device from being in the clamping state for a long period of time and deformed due to stress to cause the limiting device to become loose

The present disclosure provides a limiting device, which can realize the function of fast moving and positioning, and still can maintain a very good position limiting function after a long-time placement, thus facilitating the operation, and prolonging the service life of the limiting device.

### Brief Description of Drawings

FIG. 1 is a schematic view of a limiting device of the present disclosure;
FIG. 2a is a schematic view of the limiting device of the present disclosure in a non-clamping state;
FIG. 2b is a sectional view of the limiting device of the present disclosure in the non-clamping state at a place A-A of a core rod;
FIG. 3a is a schematic view of the limiting device of the present disclosure in a clamping state; and
FIG. 3b is a sectional view of the limiting device of the present disclosure in the clamping state at the place A-A of the core rod.

### Illustration of reference signs:

| | |
|---|---|
| 1 core rod | 2 slider |
| 3 limiting device | 3-a clamping portion |
| 3-b movable portion | 4 pressing plate |

### Detailed Description of Embodiments

In order to make the objectives, the technical solutions and advantages of the present disclosure clearer, the present disclosure is further described in detail below in combination with the accompanying drawings and the embodiments. It should be understood that the specific embodiments described herein are merely used to illustrate the present disclosure, rather than limiting the present disclosure. The scope of the present disclosure is not limited by these embodiments, but is determined by the scope of the claims.

In order to provide clearer description and enable one of ordinary skill in the art to understand the filed contents of the present disclosure, various parts within the drawings are not necessarily drawn to scale relative to each other, the ratio of certain dimensions to other related dimensions may be highlighted and exaggerated, and irrelevant or unimportant details may not be fully drawn for clarity of illustration.

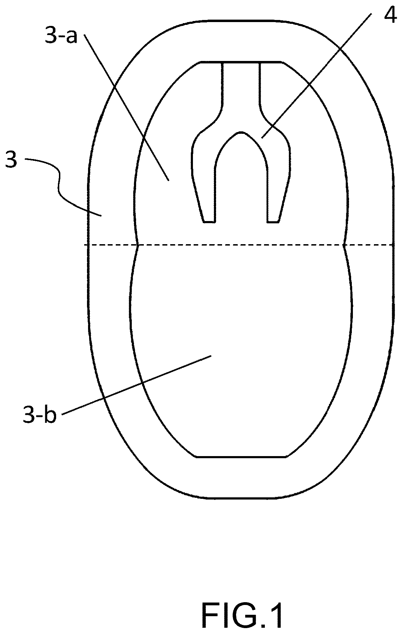

FIG. 1 to FIG. 3b are schematic views of a limiting device of the present disclosure, and schematic views of the limiting device in a clamping state and a non-clamping state when cooperating with a core rod. The limiting device 3 in the present disclosure includes a clamping portion 3-a and a movable portion 3-b. The clamping portion 3-a and the movable portion 3-b may be shaped as required, and may be respectively in a ring structure. Two ends of the clamping portion 3-a in a ring structure are respectively connected to two ends of the movable portion 3-b in a ring structure, thus forming a connected double-ring structure. In the present embodiment, the ring structures of the clamping portion and the movable portion are C-shaped structures, and two ends of the C-shaped structures of the clamping portion and the movable portion are respectively connected to each other to form the connected double-ring structure. In the above, a ring diameter of the movable portion 3-b is greater than a ring diameter of the clamping portion 3-a, and the clamping portion 3-a may cooperate with a core rod 1 to realize a limiting function of a slider 2. The slider 2 and the core rod 1 may be shaped as required. In the present embodiment, the slider 2 may be a ring-shaped slider, the core rod 1 may be in a cylindrical shape, and the slider 2 may slide on the core rod 1. The clamping portion 3-a and the core rod 1 are in an interference fit, and the movable portion 3-b and the core rod 1 are in a clearance fit. The limiting device 3 and the slider 2 are respectively arranged on the core rod 1. When the limiting device 3 does not need to limit the movement of the slider 2, the movable portion 3-b cooperates with the core rod 1, as the movable portion 3-b and the core rod 1 are in a clearance fit, at this time, the clamping portion 3-a and the core rod 1 are not in pressure contact, and even if after long-time placement, the clamping portion 3-a still will not be deformed to affect the clamping effect, thus greatly prolonging the service life of the limiting device 3, moreover, the limiting device 3 may smoothly slide on the core rod 1, when the slider 2 needs to be positioned, the slider 2 is slid to be brought into a designated position, then, the limiting device 3 is slid so that it is moved to a designated position. During this positioning process, as the movable portion 3-b and the core rod 1 are in a clearance fit, the limiting device 3 may slide freely on the core rod 1, to complete the positioning operation, thus the operation is simpler; when the limiting device 3 moves to the designated position, the clamping portion 3-a is pressed so that the clamping portion 3-a cooperates with the core rod 1. As the clamping portion 3-a and the core rod 1 of the handle are in an interference fit, the limiting device 3 cannot slide along the core rod 1, thereby achieving a position limiting effect, at this time, the ring slider 2 of the handle is fixed at this position, and can no longer move, thereby achieving the purpose of stopping through the ring slider 2. In order to achieve a better clamping effect, when the movable portion 3-b in the limiting device 3 cooperates with the core rod 1, an area of the core rod 1 wrapped by the movable portion 3-b is greater than half of a ring area of the core rod 1, and the limiting device 3 will not slide off the core rod 1; and when the clamping portion 3-a in the limiting device 3 cooperates with the core rod 1, the area of the core rod 1 wrapped by the clamping portion 3-a is greater than half of the ring area of the core rod 1, and a better fixing effect may be achieved.

In order to achieve a better stopping and position limiting effect, a fixing pressure of the limiting device 3 at a designated position is increased. In the present disclosure, a fork-shaped pressing plate 4 further may be provided in the ring of the clamping portion 3-a, and a groove that may cooperate with the fork-shaped pressing plate 4 is provided on the core rod 1, and the groove may cooperate with the fork-shaped pressing plate 4 to increase the fixing pressure of the limiting device 3 at the designated position. Specifically, when the movable portion 3-b cooperates with the core rod 1, only end portions of the fork-shaped pressing plate 4 are in contact with an edge of the groove of the core rod 1, and at this time, no pressure will be generated between the fork-shaped pressing plate 4 and the groove of the core rod 1, the fixing force of the fork-shaped pressing plate 4 will not be affected after long-time placement, thus functioning to fix the position during the sliding process of the limiting device, and preventing a sliding portion from inclining during the moving process; and when the clamping portion 3-a cooperates with the core rod 1, the fork-shaped pressing plate 4 may be mostly or completely pressed into the groove of the core rod 1. At this time, an extruding force between the fork-shaped pressing plate 4 and the groove of the core rod will increase the fixing force of the limiting device 3, and improve the positioning effect between the limiting device 3 and the core rod 1. In order to achieve a better clamping effect, the length of the pressing plate 4 may be greater than half of the depth of the groove and less than the depth of the groove. In the present disclosure, the shape of the pressing plate 4 is a fork shape, which is not limited herein. The pressing plate includes two or more teeth, and the teeth have a gap therebetween, and can be deformed correspondingly so that they are pressed into the groove of the core rod 1, with a contact surface between the pressing plate and the groove of the core rod 1 being in an arc shape, and the fixing effect is achieved. Apart from the fork shape in the present embodiment, the pressing plate may also be of a zigzag shape or other shapes.

In the present disclosure, the movable portion 3-b and the clamping portion 3-a in the limiting device 3 may be integrally formed or separately manufactured. An advantage of using a separately manufactured structure is that when the movable portion 3-b or the clamping portion 3-a fails, the failed part may be disassembled and then replaced, without affecting the use of other parts, thereby facilitating the operation. When the movable portion 3-b and the clamping portion 3-a in the limiting device 3 adopt discrete structures, the movable portion 3-b and the clamping portion 3-a may be fixed by glue, hook, or the like, or may be fixed in a plurality of mixed manners.

In the present disclosure, the material of the limiting device 3 is plastic, and the limiting device 3 is formed by injection molding. The plastic herein may be ABS, PC or other plastic materials, and may also be a mixed material composed of a plurality of plastic raw materials. The selected material should have good dimensional stability, and have certain toughness and rigidity.

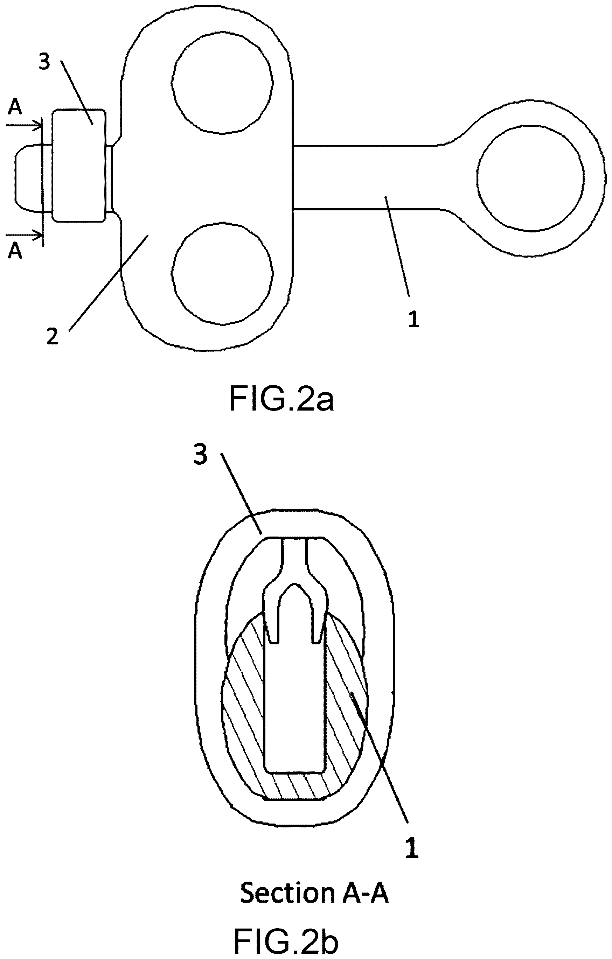

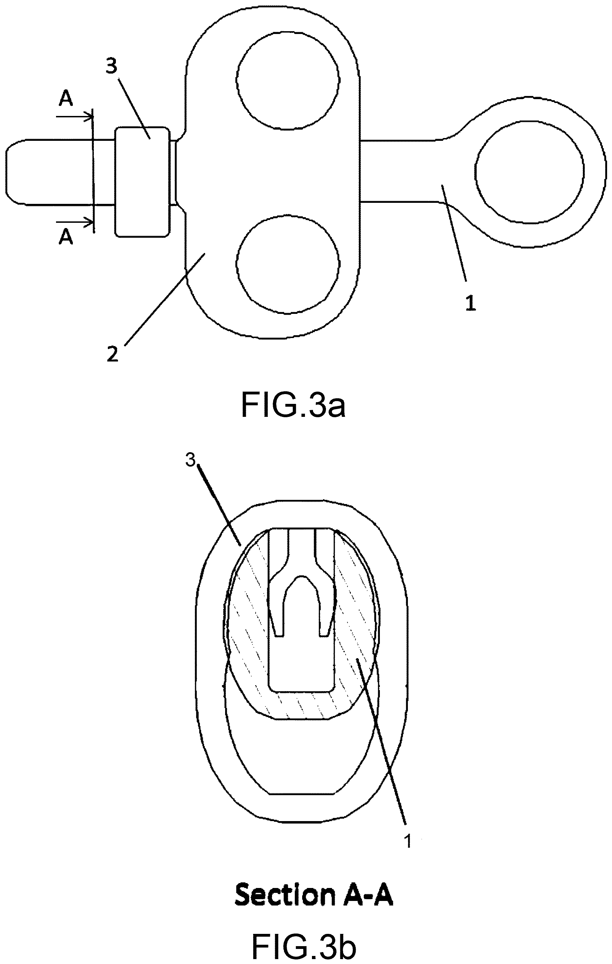

FIG. 2a and FIG. 3a respectively show schematic views of the limiting device 3 of the present disclosure, when cooperating with the core rod 1, in a clamping state and a non-clamping state, and FIG. 2b and FIG. 3b respectively show sectional views of the limiting device 3 of the present disclosure at a place A-A of the core rod, when cooperating with the core rod 1 in a clamping state and a non-clamping state. As shown in FIG. 2b, at this time, the limiting device 3 is in a non-clamping state, and the movable portion 3-b in the limiting device cooperates with the core rod 1, and the area of the core rod 1 wrapped by the movable portion is greater than half of the ring area of the core rod, moreover, as the movable portion 3-b and the core rod 1 are in a clearance fit, the limiting device 3 may slide freely on the core rod 1. When the instrument is in an unused state, the limiting device 3 is in a non-clamping state, so that no pressure will be generated on the limiting device 3, and the limiting device will not be deformed even after long-time placement; when the instrument is in a use state and the position of the limiting device 3 needs to be adjusted, the limiting device 3 only needs to be pushed to a designated position, which facilitates operation and reduces the risk of wear fatigue of the limiting device 3. As shown in FIG. 3b, at this time, the limiting device 3 is in a clamping state, at this time, the clamping portion 3-a in the limiting device 3 cooperates with the core rod 1, the pressing plate 4 in the clamping portion 3-a is mostly or completely pressed into the groove of the core rod 1. As the clamping portion 3-a and the core rod 1, and the pressing plate 4 and the groove of the core rod 1 are in an interference fit, respectively, there is a large clamping force between the limiting device 3 and the core rod 1 at this time, then the limiting device 3 cannot slide along the core rod 1 of the handle, thus achieving the purpose of limiting the movement of the slider 2.

The main function of the present disclosure in the ERCP is as follows: after a papillotome reaches a designated position, the doctor pulls the ring slider on the handle, so that cutting wire at a distal end of the papillotome is adjusted to be in a bow shape suitable for incising papilla. At this time, the movable portion in the limiting device and the core rod are in a clearance fit state, the limiting device is slid to a front end of the ring slider, and presses the clamping portion of the limiting device, so that the clamping portion in the limiting device cooperates with the core rod of the handle. At this time, as the clamping portion and the core rod of the handle are in an interference fit, the limiting device will be in a clamping state, it cannot slide along the core rod of the handle, then having a limiting function to the ring slider; when there is no need to limit the position, the movable portion of the limiting device is pressed, so that the movable portion cooperates with the core rod of the handle, thereby avoiding the clamping force of the limiting device from being reduced when the limiting device is in the clamping state for a long time, thereby the limiting function cannot be realized.

The limiting device provided in the present disclosure can realize the function of fast moving and positioning, and still can maintain a very good limiting function after long-time placement, thus prolonging the service life of the medical device.

The above-mentioned are merely preferred embodiments of the present disclosure, but do not limit the present disclosure in any form. Although the present disclosure has been disclosed in the above with preferred embodiments, the present disclosure is not limited thereto.

## Claims

1. A position limiting device (3) configured to be used in a surgical device, comprising a clamping portion (3-a) and a movable portion (3-b), **characterized in that** the clamping portion (3-a) and the movable portion (3-b) are both of an arc structure, two ends of the clamping portion (3-a) in the arc structure and two ends of the movable portion (3-b) in the arc structure are connected, respectively, forming a connected double-ring structure; a ring diameter of the movable portion is greater than a ring diameter of the clamping portion; and the position limiting device (3) is provided on a core rod (1), the clamping portion (3-a) and the core rod (1) are in an interference fit, and the movable portion (3-b) and the core rod (1) are in a clearance fit, wherein when the movable portion (3-b) cooperates with the core rod (1), the position limiting device (3) is slidable with respect to the core rod (1), and when the clamping portion (3-a) cooperates with the core rod (1), the position limiting device (3) and the core rod (1) are in a fixed state, and the position limiting device (3) is immovable with respect to the core rod (1), thus achieving a position limiting purpose.

2. The position limiting device (3) according to claim 1, wherein the core rod (1) is further provided with a slider (2), wherein when the slider (2) moves to a designated position, to enable the clamping portion (3-a) to cooperate with the core rod (1), the position limiting device (3) and the core rod (1) are in a fixed state, so that the position limiting device (3) limits movement of the slider (2).

3. The position limiting device (3) according to claim 1, wherein when the clamping portion (3-a) and the movable portion (3-b) cooperate with the core rod (1), an area of the core rod (1) wrapped by each of the clamping portion (3-a) and the movable portion (3-b) is greater than half of a ring area of the core rod (1).

4. The position limiting device (3) according to claim 1, wherein the clamping portion (3-a) is provided with a pressing plate (4), and the core rod (1) is provided with a groove cooperating with the pressing plate (4).

5. The position limiting device (3) according to claim 4, wherein the pressing plate (4) is of a fork shape or a zigzag shape.

6. The position limiting device (3) according to claim 4, wherein a depth of the groove is greater than a length of the pressing plate (4).

7. The position limiting device (3) according to claim 6, wherein when the movable portion (3-b) cooperates with the core rod (1), the position limiting device (3) is in a non-clamping state, and only end portions of the pressing plate (4) are in contact with the groove of the core rod (1); and when the clamping portion (3-a) cooperates with the core rod (1), the position limiting device (3) is in a clamping state, the pressing plate (4) is mostly or completely pressed into the groove of the core rod (1), and the position limiting device (3) is immovable with respect to the core rod (1).

8. The position limiting device (3) according to claim 1, wherein the clamping portion (3-a) and the movable portion (3-b) are integrally molded or separately molded.

9. The position limiting device according to claim 1, wherein the arc structure of each of the clamping portion (3-a) and the movable portion (3-b) is a C-shaped structure.

10. The position limiting device according to any one of claims 1-9, wherein the position limiting device (3) is made of plastic.

11. Method for using the position limiting device (3) according to claim 1, **characterized in that** when the position limiting device (3) is in a non-clamping state, the position limiting device (3) is slid to a designated portion; the clamping portion (3-a) of the position limiting device (3) is pressed, so that the clamping portion (3-a) of the position limiting device (3) cooperates with the core rod (1), and at this time, the position limiting device (3) is in a clamping state; and after a position limiting is completed, the movable portion (3-b) of the position limiting device (3) is pressed, so that the movable portion (3-b) of the position limiting device (3) cooperates with the core rod (1), the position limiting device (3) is again in the non-clamping state, avoiding the core rod (1) and the position limiting device (3) from being in the clamping state for a long period of time and deformed due to stress to cause the position limiting device (3) to become loose.

## Patentansprüche

1. Positionsbegrenzungsvorrichtung (3), die konfiguriert ist, in einer chirurgischen Vorrichtung verwendet zu werden, umfassend einen Klemmabschnitt (3-a) und einen beweglichen Abschnitt (3-b), **dadurch gekennzeichnet, dass** der Klemmabschnitt (3-a) und der bewegliche Abschnitt (3-b) beide eine Bogenstruktur aufweisen, zwei Enden des Klemmabschnitts (3-a) in der Bogenstruktur und zwei Enden des beweglichen Abschnitts (3-b) in der Bogenstruktur jeweils verbunden sind, wobei sie eine verbundene Doppelringstruktur bilden; ein Ringdurchmesser des beweglichen Abschnitts größer ist als ein Ringdurchmesser des Klemmabschnitts; und die Positionsbegrenzungsvorrichtung (3) an einer Kernstange (1) bereitgestellt ist, der Klemmabschnitt (3-a) und die Kernstange (1) in einer Presspassung sind und der bewegliche Abschnitt (3-b) und die Kernstange (1) in einer Spielpassung sind, wobei, wenn der bewegliche Abschnitt (3-b) mit der Kernstange (1) zusammenwirkt, die Positionsbegrenzungsvorrichtung (3) in Bezug auf die Kernstange (1) verschiebbar ist, und wenn der Klemmabschnitt (3-a) mit der Kernstange (1) zusammenwirkt, die Positionsbegrenzungsvorrichtung (3) und die Kernstange (1) in einem fixierten bzw. festen Zustand sind und die Positionsbegrenzungsvorrichtung (3) in Bezug auf die Kernstange (1) unbeweglich ist, wodurch ein Positionsbegrenzungszweck erfüllt ist.

2. Positionsbegrenzungsvorrichtung (3) nach Anspruch 1, wobei die Kernstange (1) ferner mit einem Schieber (2) versehen ist, wobei, wenn sich der Schieber (2) zu einer festgelegten Position bewegt, um dem Klemmabschnitt (3-a) zu ermöglichen, mit dem Kernstange (1) zusammenzuwirken, die Positionsbegrenzungsvorrichtung (3) und die Kernstange (1) in einem fixierten Zustand sind, so dass die Positionsbegrenzungsvorrichtung (3) die Bewegung des Schiebers (2) begrenzt.

3. Positionsbegrenzungsvorrichtung (3) nach Anspruch 1, wobei, wenn der Klemmabschnitt (3-a) und der bewegliche Abschnitt (3-b) mit der Kernstange (1) zusammenwirken, ein Bereich der Kernstange (1), der von jedem des Klemmabschnitts (3-a) und des beweglichen Abschnitts (3-b) umgeben ist, größer ist als die Hälfte einer Ringfläche der Kernstange (1).

4. Positionsbegrenzungsvorrichtung (3) nach Anspruch 1, wobei der Klemmabschnitt (3-a) mit einer Pressplatte (4) versehen ist und die Kernstange (1) mit einer Nut versehen ist, die mit der Pressplatte (4) zusammenwirkt.

5. Positionsbegrenzungsvorrichtung (3) nach Anspruch 4, wobei die Pressplatte (4) eine Gabelform oder eine Zick-Zack-Form aufweist.

6. Positionsbegrenzungsvorrichtung (3) nach Anspruch 4, wobei eine Tiefe der Nut größer ist als eine Länge der Pressplatte (4).

7. Positionsbegrenzungsvorrichtung (3) nach Anspruch 6, wobei, wenn der bewegliche Abschnitt (3-b) mit dem Kernstange (1) zusammenwirkt, sich die Positionsbegrenzungsvorrichtung (3) in einem Nichtklemmzustand befindet und nur Endabschnitte der Pressplatte (4) mit der Nut der Kernstange (1) in Kontakt sind; und wenn der Klemmabschnitt (3-a) mit der Kernstange (1) zusammenwirkt, sich die Positionsbegrenzungsvorrichtung (3) in einem Klemmzustand befindet, die Pressplatte (4) größtenteils oder vollständig in die Nut der Kernstange (1) gepresst ist und die Positionsbegrenzungsvorrichtung (3) in Bezug auf die Kernstange (1) unbeweglich ist.

8. Positionsbegrenzungsvorrichtung (3) nach Anspruch 1, wobei der Klemmabschnitt (3-a) und der bewegliche Abschnitt (3-b) integral bzw. einstückig geformt oder separat geformt sind.

9. Positionsbegrenzungsvorrichtung nach Anspruch 1, wobei die Bogenstruktur jedes des Klemmabschnitts (3-a) und des beweglichen Abschnitts (3-b) eine C-förmige Struktur ist.

10. Positionsbegrenzungsvorrichtung nach einem der Ansprüche 1-9, wobei die Positionsbegrenzungsvorrichtung (3) aus Kunststoff besteht.

11. Verfahren zum Verwenden der Positionsbegrenzungsvorrichtung (3) nach Anspruch 1, **dadurch gekennzeichnet, dass**, wenn sich die Positionsbegrenzungsvorrichtung (3) in einem Nichtklemmzustand befindet, die Positionsbegrenzungsvorrichtung (3) zu einem festgelegten Abschnitt verschoben wird; der Klemmabschnitt (3-a) der Positionsbegrenzungsvorrichtung (3) gepresst wird, so dass der Klemmabschnitt (3-a) der Positionsbegrenzungsvorrichtung (3) mit der Kernstange (1) zusammenwirkt, und sich die Positionsbegrenzungsvorrichtung (3) zu diesem Zeitpunkt in einem Klemmzustand befindet; und nachdem eine Positionsbegrenzung abgeschlossen ist, der bewegliche Abschnitt (3-b) der Positionsbegrenzungsvorrichtung (3) gepresst wird, so dass der bewegliche Abschnitt (3-b) der Positionsbegrenzungsvorrichtung (3) mit der Kernstange zusammenwirkt (1), sich die Positionsbegrenzungsvorrichtung (3) wieder in dem Nichtklemmzustand befindet, wodurch vermieden wird, dass sich die Kernstange (1) und die Positionsbegrenzungsvorrichtung (3) über einen längeren Zeitraum in dem Klemmzustand befinden und durch Belastung verform werden, um zu bewirken bzw. was bewirkt, dass sich die Positionsbegrenzung (3) lockert.

## Revendications

1. Dispositif de limitation de position (3) configuré pour être utilisé dans un dispositif chirurgical, comprenant une partie de serrage (3-a) et une partie mobile (3-b), **caractérisé en ce que** la partie de serrage (3-a) et la partie mobile (3-b) sont toutes les deux de structure arquée, deux extrémités de la partie de serrage (3-a) dans la structure arquée et deux extrémités de la partie mobile (3-b) dans la structure arquée sont reliées, respectivement, formant une structure à deux anneaux reliée ; un diamètre d'anneau de la partie mobile est plus grand qu'un diamètre d'anneau de la partie de serrage ; et le dispositif de limitation de position (3) est prévu sur une tige centrale (1), la partie de serrage (3-a) et la tige centrale (1) sont dans un ajustement serré, et la partie mobile (3-b) et la tige centrale (1) sont dans un ajustement avec jeu, dans lequel lorsque la partie mobile (3-b) coopère avec la tige centrale (1), le dispositif de limitation de position (3) peut coulisser par rapport à la tige centrale (1), et lorsque la partie de serrage (3-a) coopère avec la tige centrale (1), le dispositif de limitation de position (3) et la tige centrale (1) sont dans un état fixe, et le dispositif de limitation de position (3) est immobile par rapport à la tige centrale (1), obtenant ainsi un objectif de limitation de position.

2. Dispositif de limitation de position (3) selon la revendication 1, dans lequel la tige centrale (1) est en outre pourvue d'un coulisseau (2), dans lequel lorsque le coulisseau (2) se déplace vers une position désignée, pour permettre à la partie de serrage (3-a) de coopérer avec la tige centrale (1), le dispositif de limitation de position (3) et la tige centrale (1) sont dans un état fixe, de sorte que le dispositif de limitation de position (3) limite le déplacement du coulisseau (2).

3. Dispositif de limitation de position (3) selon la revendication 1, dans lequel lorsque la partie de serrage (3-a) et la partie mobile (3-b) coopèrent avec la tige centrale (1), une superficie de la tige centrale (1) enveloppée par chacune de la partie de serrage (3-a) et la partie mobile (3-b) est plus grande que la moitié d'une superficie annulaire de la tige centrale (1).

4. Dispositif de limitation de position (3) selon la revendication 1, dans lequel la partie de serrage (3-a) est pourvue d'une plaque de pressage (4), et la tige centrale (1) est pourvue d'une rainure coopérant avec la plaque de pressage (4).

5. Dispositif de limitation de position (3) selon la revendication 4, dans lequel la plaque de pressage (4) est en forme de fourchette ou en forme de zigzag.

6. Dispositif de limitation de position (3) selon la revendication 4, dans lequel une profondeur de la rainure est plus grande qu'une longueur de la plaque de pressage (4).

7. Dispositif de limitation de position (3) selon la revendication 6, dans lequel lorsque la partie mobile (3-b) coopère avec la tige centrale (1), le dispositif de limitation de position (3) est dans un état de non-serrage, et seules les parties d'extrémité de la plaque de pressage (4) sont en contact avec la rainure de la tige centrale (1) ; et lorsque la partie de serrage (3-a) coopère avec la tige centrale (1), le dispositif de limitation de position (3) est dans un état de serrage, la plaque de pressage (4) est en grande partie ou complètement pressée dans la rainure de la tige centrale (1), et le dispositif de limitation de position (3) est immobile par rapport à la tige centrale (1).

8. Dispositif de limitation de position (3) selon la revendication 1, dans lequel la partie de serrage (3-a) et la partie mobile (3-b) sont moulées d'un seul tenant ou moulées séparément.

9. Dispositif de limitation de position selon la revendication 1, dans lequel la structure arquée de chacune de la partie de serrage (3-a) et la partie mobile (3-b) est une structure en forme de C.

10. Dispositif de limitation de position selon l'une quelconque des revendications 1-9, dans lequel le dispositif de limitation de position (3) est en plastique.

11. Procédé pour utiliser le dispositif de limitation de position (3) selon la revendication 1, **caractérisé en ce que** lorsque le dispositif de limitation de position (3) est dans un état de non-serrage, le dispositif de limitation de position (3) est amené à coulisser vers une partie désignée ; la partie de serrage (3-a) du dispositif de limitation de position (3) est pressée, de sorte que la partie de serrage (3-a) du dispositif de limitation de position (3) coopère avec la tige centrale (1), et à ce moment, le dispositif de limitation de position (3) est dans un état de serrage ; et après qu'une limitation de position est achevée, la partie mobile (3-b) du dispositif de limitation de position (3) est pressée, de sorte que la partie mobile (3-b) du dispositif de limitation de position (3) coopère avec la tige centrale (1), le dispositif de limitation de position (3) est à nouveau dans l'état de non-serrage, évitant à la tige centrale (1) et au dispositif de limitation de position (3) d'être dans l'état de serrage pendant une longue période de temps et déformée en raison d'une contrainte pour amener le dispositif de limitation de position (3) à se desserrer.
